# EUROPEAN PATENT APPLICATION

(11) **EP 1 090 655 A1**
(43) Date of publication of application: **11.04.2001**
(21) Application number: 00203077.3
(22) Date of filing: 08.09.2000
(51) Int. Cl.: A61M 16/20, F16K 31/528

(54) **Device for the adjustment of the flow of a gas**

(30) Priority: 09.09.1999 NL 1013009
(71) Applicant: Proclin (International) B.V., 2718 RZ Zoetermeer (NL)
(72) Inventor: Van de Graaff, Marco, 2261 ER Leidschendam (NL)
(74) Representative: De Hoop, Eric

(57) **Abstract**

Volume selector as adjustment device for dosing a wanted quantity of gas. The volume selector comprises a valve seat and a valve, the valve seat and the valve being designed movable with respect to each other. The valve is provided with a calibrated protruding part and the valve seat is provided with a calibrated opening for accommodating the protruding part therein over an adjustable distance. The valve is moveable in a linear manner, for instance by means of rotation, with respect to the valve seat, or vice versa. The degree of movement defines the adjustable distance of the protruding part in the opening. The adjusted wanted quantity of gas through the volume selector is directly related to the degree of rotation of the selector button and therefore to the degree of rotation of the valve. In the described adjustment the protruding part of the valve is narrowed and extends into a cylindrical opening. The same result can be obtained by extending a cylindrical protruding part into a narrowing opening.

## Description

The invention relates to an adjustment device, also called volume selector, for dosing a wanted quantity of gas, for instance oxygen, which adjustment device comprises a valve seat and a valve, the valve seat and the valve being designed movable with respect to each other, the valve being provided with a protruding part and the valve seat being provided with an opening for accommodating the protruding part of the valve therein, over an adjustable distance, the valve being arranged in a linear moveable manner, for instance by rotation, with respect to the valve seat, or vice versa, the degree of movement of the valve or valve seat , for instance the degree of rotation, defining the adjustable distance of the protruding part in the opening.

In the industry, for dosing a wanted quantity of gas, for instance oxygen, amongst others a combination of a needle valve, for adjusting a gas flow, with a connected separate gas flow meter on which the quantity of gas flown through can be read, is known. Said principle is for instance mentioned in FR 2 645 027. In said document a device is described, which comprises both an adjusting valve and a gas flow meter for adjusting the supply of gas, for instance oxygen, for medicinal uses. The valve from FR 2 645 027 comprises a conical, protruding part, which is accommodated in an opening over an adjustable distance. Said opening is cylindrical. The protruding part of the valve is designed such that it narrows in the direction of the opening. By varying the adjustable distance the protruding part of the valve is extended to higher or lesser degree into the opening, as a result of which the slit between the conical protruding part of the valve and the opening will vary in cross-section.

As a result the gas flow between the conical protruding part and the opening will also vary when the pressure of the gas in the opening remains the same. In this way the gas flow can be adjusted with the adjustment device from FR 2 645 027, but the size of the gas flow as such is unknown. The separate gas flow meter is designed as an elongated tube in a vertical position, through which the gas flow is led in upward direction. In said elongated vertical tube a ball is present which can move freely in the elongated vertical tube. When gas flows through the elongated vertical tube the ball will be moved in upward direction by the pressure of the gas. Because the elongated vertical tube widens in upward direction, the width of the slit between the ball and the inner side of the elongated vertical tube will increase in upward direction. As a result ever more gas flow is needed to move the ball to a higher position in the elongated vertical tube. Because a calibration has been made on the elongated vertical tube and because it is transparent the quantity of gas flowing through can be read from it. A drawback of the known adjustment device with gas flow meter is that it always has to be kept straight up and as such is less suitable for use under moving conditions, such as for instance in a riding ambulance. As a result of the shaking while the ambulance is being driven, the ball will after all move up and down in vertical direction in the elongated vertical tube which makes it difficult to read and therefore adjust the correct quantity of gas.

It is an object of the invention to provide an adjustment device, in which, also in moving conditions, no use has to be made of a sensitive gas flow meter, so that a correct adjustment and reading of the wanted quantity of gas is possible in for instance a riding ambulance or a deviating mounting position of the adjustment device.

To that end according to one aspect of the invention an adjustment device is provided for adjusting the quantity of gas, for instance oxygen, which adjustment device comprises a valve seat and a valve, the valve seat and the valve being designed movable with respect to each other, the valve being provided with a protruding part and the valve seat being provided with an opening for accommodating the protruding part therein, over an adjustable distance, the valve being arranged in a moveable manner with respect to the valve seat by means of rotation, the degree of rotation defining the adjustable distance of the protruding part in the opening and an adjusted quantity of gas, and in which the adjusted quantity of the adjustment device can directly be read from the degree of rotation. This results in an adjustment device in which the adjustment and reading of the quantity of gas can take place correctly independent from the movements, such as for instance during riding an ambulance.

A further embodiment of the adjustment device according to the invention comprises a housing, in which the valve seat can be immovably attached to the housing, in which the housing comprises an entrance and an exit for the gas and in which the valve is arranged in the housing in a rotatable manner. By arranging the valve in the housing in a rotatable manner the position of the valve with respect to the valve seat and the housing can be changed in an exact and reproducible manner.

The quantity of gas of the adjustment device according to the invention can be adjusted in an advantageous manner when the valve has an exterior surface which is provided with a helical groove, and when an adjusting screw can be screwed through the housing so that one of its ends extends into the groove in an unsqueezing manner. By rotating the valve the groove will move along the screw and the valve will translate with respect to the housing.

Rotating the valve with respect to the housing and the valve seat of the adjustment device according to the invention for adjusting the quantity of gas may take place if the valve comprises a rear part protruding from the housing to which part a selector button can be attached, on which selector button a calibration has been arranged. The selector button has a casing which envelops the adjusting screw which extends into the groove of the valve. Through the selector button the valve can be rotated with respect to the valve seat and the housing.

Repairs and replacement of parts can take place quickly and easy, when the adjustment device according to the invention is designed in a modular manner.

Preferably the protruding part of the valve is conically narrowing in the direction of the opening, preferably at a gradient of 1:25 or milder, in particular approximately 1:50, in which the width of the end plane of the tip is merely a few mm, for instance 2 mm.

A practical advantageous embodiment according to the invention is provided by an adjustment device for adjusting the quantity of a gas, for instance oxygen, consisting of:
a valve and a valve seat, the valve seat and the valve being designed movable with respect to each other, the valve being provided with a protruding part and the valve seat being provided with an opening for accommodating the protruding part therein over an adjustable distance, the valve being moveable with respect to the valve seat by means of rotation, the degree of rotation defining the adjustable distance of the protruding part in the opening and an adjusted quantity of a gas,
a housing, in which the valve seat can be immovably attached to the housing and in which the housing comprises an entrance and an exit for the gas and in which the valve is arranged in the housing in a rotatable manner, the valve having an exterior surface which is provided with a helical groove,
a screw that can be screwed through the housing so that one of its ends extends into the groove,
the valve comprising a part protruding from the housing to which part a selector button can be attached, on which selector button a calibration has been arranged, so that the adjusted quantity of the adjustment device can directly be read from the degree of rotation of the valve, the selector button having a casing which envelops the screw,
an inlet cap and a filter,
an O-ring between the inlet cap and the housing,
an O-ring between the valve seat and the housing,
an O-ring between the valve and the housing above the groove in the valve and an O-ring between the valve and the housing under the groove in the valve,
and in which the protruding part of the valve is conical, narrowing in the direction of the opening. Such an adjustment device solely consisting of the parts mentioned provides a reproducible and accurately readable adjustment of the wanted adjusted quantity of gas.

According to a further aspect of the invention a volume selector is provided for dosing a wanted adjusted quantity of gas, for instance oxygen, which volume selector comprises a valve seat and a valve, the valve seat and the valve being designed movable with respect to each other, the valve being provided with a calibrated protruding part and the valve seat being provided with a calibrated opening for accommodating the protruding part therein, over an adjustable distance, the valve being arranged in a linear moveable manner, for instance by rotation, with respect to the valve seat, the degree of linear movement defining the adjustable distance of the protruding part in the opening, the wanted adjusted quantity of gas through the volume selector being directly related to the degree of linear movement of the valve, for instance to the degree of rotation of a calibrated selector button, without an additional passage flow meter being necessary.

A further embodiment of the invention provides that the volume selector comprises a housing, in which the valve seat can be immovably attached to the housing, in which the housing comprises an entrance and an exit for the gas flow and that the valve is arranged in the housing in a linear moveable manner, for instance by rotation.

According to a further aspect of the invention it is provided that the valve in the practical embodiment has an exterior surface which is provided with a helical groove, and that the housing is provided with an adjusting screw of which the end extends into the helical groove of the valve in an unsqueezing manner,
that the valve comprises a rear part protruding from the housing to which part a selector button can be attached, on which selector button a calibration has been arranged,
that the selector button has a casing which envelops the adjusting screw which extends into the groove of the valve, so that the volume selector cannot be taken out just like that,
that the volume selector comprises an inlet cap with a filter,
that the volume selector comprises an O-ring between the inlet cap and the housing,
that the volume selector comprises an O-ring between the valve seat and the housing,
that the volume selector comprises an O-ring between the valve and the housing above the groove in the valve and comprises an O-ring between the valve and the housing under the groove in the valve,
that the calibrated protruding part of the valve is conically narrowing in the direction of the opening,
that the opening of the valve seat has been calibrated,
that the thickness variation of the conical protruding part has been calibrated,
that the material of all main parts is stainless steel,
that the volume selector is designed in a modular manner.

The invention also provides a volume selector for dosing the wanted adjusted quantity of gas, for instance oxygen, consisting of: a valve and a valve seat, the valve seat and the valve being designed movable with respect to each other, the valve being provided with a protruding part and the valve seat being provided with an opening for accommodating the protruding part therein over an adjustable distance, the valve being arranged in a linear moveable manner with respect to the valve seat by means of rotation, the degree of movement defining the adjustable distance of the protruding part in the opening, a housing, in which the valve seat can be immovably attached to the housing and in which the housing comprises an entrance and an exit for the gas flow and in which the valve is arranged in the housing in a linear moveable manner, the valve having an exterior surface which is provided with a calibrated helical groove, an adjusting screw which is screwed in the housing so that one of its ends extends into the groove of the valve, the valve having a rear part protruding from the housing to which part a selector button can be attached, on which selector button a calibration has been arranged, so that the wanted adjusted quantity of gas through the volume selector is directly related to the degree of rotation of the valve, the selector button having a casing which envelops the adjusting screw, an inlet cap and a filter, an O-ring between the inlet cap and the housing, an O-ring between the valve seat and the housing, an O-ring between the valve and the housing above the groove in the valve and an O-ring between the valve and the housing under the groove in the valve, and in which the calibrated protruding part of the valve is conical, narrowing in the direction of the calibrated opening.

According to a further aspect of the invention a volume selector is provided for dosing the wanted adjusted quantity of gas, for instance oxygen, which volume selector comprises a valve seat and a valve, the valve seat and the valve being designed movable with respect to each other, the valve seat being provided with a protruding part and the valve being provided with an opening for accommodating the protruding part therein, over an adjustable distance, the valve being arranged in a linear moveable manner with respect to the valve seat, the degree of movement defining the adjustable distance of the protruding part in the opening, the wanted adjusted quantity of gas through the volume selector being directly related to the degree of rotation of the valve and thus to the rotation of the calibrated selector button.

Below some exemplary embodiments of the invention will be described by way of example on the basis of the drawings.

Figure 1 is the schematic cross-section of an exemplary embodiment of a volume selector according to the invention in an open position.

Figure 2 is a schematic cross-section of an exemplary embodiment of a volume selector according to the invention in closed position.

Figure 3 shows an arrangement of calibration of the adjustment button of the volume selector of the figures 1 and 2.

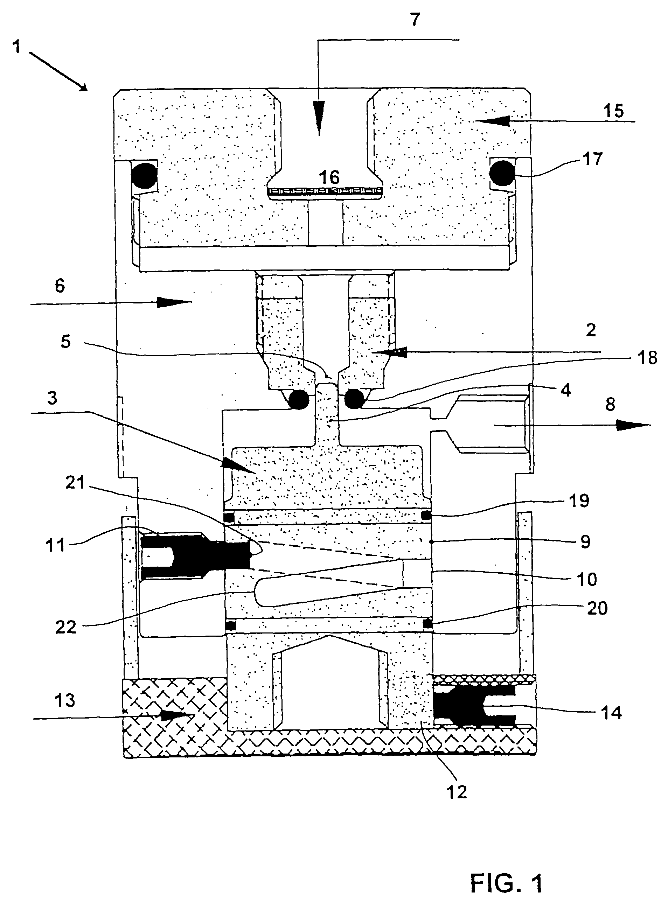

Figure 1 shows a schematic cross-section of an exemplary embodiment of the volume selector 1 according to the invention in open position. The volume selector 1 is used for dosing the wanted quantity of gas, for instance oxygen, in particular for medical uses.

The volume selector 1 has a valve seat 2 and a valve 3, the valve seat 2 and the valve 3 being designed in a moveable manner with respect to each other. The valve 3 is provided with a protruding part 4 and the valve seat 2 is provided with an opening 5 for accommodating the protruding part 4 therein over an adjustable distance. The valve 3 is arranged in a linear moveable manner with respect to the valve seat 2. The protruding part 4 of the valve 3 is conical narrowing in the direction of the opening 5. The thickness variation of the conical protruding part 4 has been accurately calibrated. The gradient of the cone is milder than 1:25, here approximately 1:50, with a taper of 0.09 over 5 mm length. The width of the tip end here for instance is 1.9 mm, in which the projection conically widens at said angle to more than 2 mm, in order to be able to close off the opening 5 having a diameter of 2 mm at a location of a diameter of 2 mm.

By altering the distance over which the protruding part 4 is accommodated in the opening 5, the width of the slit between the protruding part 4 and the opening 5 is altered. As a result the gas flow through the slit will be altered when the pressure of the gas in the opening remains unaltered. Because of the calibration the dosing of the correct quantity of gas is guaranteed, each adjustment of the selector button will let a volume of gas pass through, which is known to the user.

The valve from figure 1 is in the open position. The gas flow through the volume selector 1 now is at a maximum. It is noted that in figure 1 no slit can be seen between the opening 5 and the protruding part 4. This is because this example regards small and accurately calibrated dimensions. In this position of the valve 3 a slit, which is not shown, is most certainly present.

The volume selector 1 comprises a housing 6, in which the valve seat 2 is attached to the housing 6 in an immoveable detachable manner through a screw connection. The housing 6 has an entrance 7 and an exit 8 for the gas flow. Because the valve 3 is arranged in a linear moveable manner with respect to the housing 6 and because the valve seat 2 and the housing 6 are immovably connected to each other, the valve 3 can also be moved in a linear manner with respect to the valve seat 2.

The valve 3 has an external surface 9 which is provided with an accurately calibrated helical groove 10. An adjusting screw 11 is screwed through the housing 6 so that one of its ends extends into the groove 10 of the valve fitting snugly yet not in a squeezing manner. Because of the groove 10 and the adjusting screw 11 it is possible to move the valve 3 with respect to the housing 6 in a linear manner. After all, when the valve 3 is rotated it will move in a linear manner through the adjusting screw 11 arranged in the groove 10, with respect to the housing 6. The degree of rotation and therefore the degree of linear movement defines the adjustable distance of the protruding part 4 in the opening 5. The wanted quantity of gas through the volume selector 1 is, according to the invention, due to the calibrated groove 10, the calibrated opening 5 in the valve seat and the calibrated protruding part 4 of the valve 3 directly related to the degree of rotation of the valve 3 and therefore to the rotation and position of the selector button 13.

Manufacture is facilitated because the groove 11 extends in the wall of the housing 6 and the groove cooperating with it has been arranged on the valve 3. Leakage flows between the moving parts can be prevented easily in such a manner.

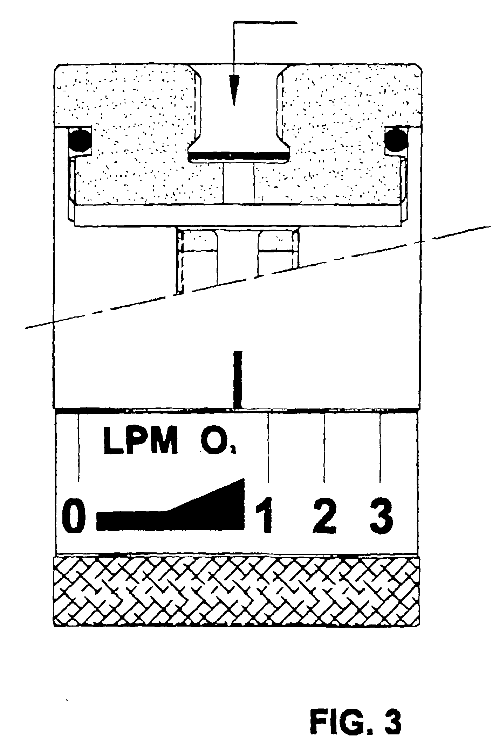

The valve 3 comprises a rear part 12 protruding from the housing 6, to which part a selector button 13 is attached. The selector button 13 has a casing through which an adjusting screw with cone 14 is screwed which is screwed in the protruding rear part 12 of the valve. Because of this adjusting screw 14 the selector button 13 is coupled to the part 12 of the valve 3. By turning the selector button 13 the valve 3 is rotated and moved in a linear manner and the wanted quantity of gas can be adjusted. On the selector button 13 a calibration has been arranged on which the adjusted quantity of gas can be read directly. Said calibration is shown in figure 3, in which it is noted that the line on the housing 6 continues until under the selector button, which itself is provided with a calibration arranged in circumferential direction related to the flow rate in l/min.

The volume selector 1 further has an inlet cap 15 having a filter 16. The function of the filter 16 is to catch unwanted particles of dirt, which would otherwise pollute the valve construction and the oxygen flow to the patient. In order to prevent leakage of gas in or from the volume selector 1 it comprises four O-rings 17, 18, 19, 20: an O-ring 17 between the inlet cap 15 and the housing 6, an O-ring 18 between the valve seat 2 and the housing 6, an O-ring 19 between the valve 3 and the housing 6 above the groove 10 in the valve 3 and an O-ring 20 between the valve 3 and the housing 6 under the groove 10 in the valve 3. Sealing of the gas flow through the volume selector 1 as long as the valve 3 is in the closed position is moreover ensured by the O-ring 18 between the housing 6 and the valve seat 2.

As appears from figure 1 the build-up of the volume selector is modular. As a result repairs and maintenance can be carried out quickly and easily.

It also appears from figure 1 that the volume selector 1 is suitable for use under bumpy or moving conditions. By bumping or movement in for instance an ambulance the position of the valve 3 will after all not be affected, as a result of which the quantity of gas to be dosed remains correctly adjustable and readable. In this way the mounting position of the volume selector 1 does not affect the adjusted gas flow either.

The volume selector 1 is designed to such a fitting extend that the movement of the valve with respect of the valve seat is free from hysteresis.

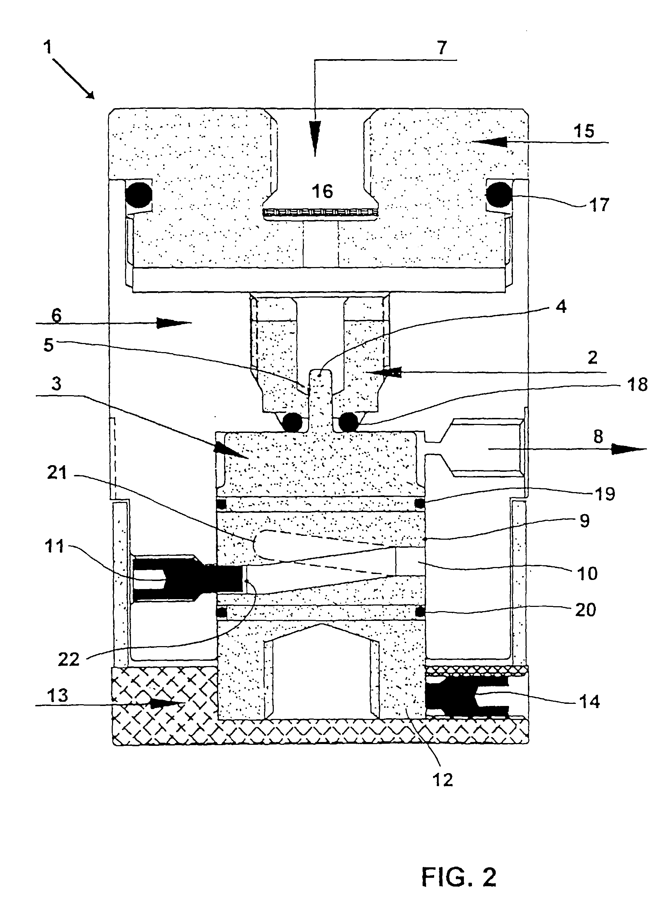

In figure 2 the same volume selector 1 as in figure 1 can be seen, however, now in the closed position. The valve 3 is now moved such through rotation, that the adjusting screw 11 is positioned in the lower end 22 of the groove 10 of the valve 3. The sealing of the slit between the protruding part 4 of the valve 3 and the opening 5 is obtained on the one hand by the abutment of the valve 3 against the O-ring 18, on the other hand by the metal-to-metal contact of the protruding part 4 against the valve seat 2, which gives an additional security in situations which entail danger of fire.

The invention further provides a volume selector for dosing a quantity of gas, for instance oxygen, which volume selector comprises a valve seat and a valve, the valve seat and the valve being designed movable with respect to each other, the valve seat being provided with a protruding part and the valve being provided with an opening for accommodating the protruding part therein over an adjustable distance. In this case the valve is arranged in a moveable manner with respect to the valve seat, or vice versa, the degree of controlled movement defining the adjustable distance of the protruding part in the opening. The adjusted quantity of gas through the volume selector is directly related to the degree of rotation of the valve and thus to the rotation of the calibrated selector button. It is noted that the movement of the valve with respect to the valve seat is activated by turning the selector button, but the movement of the valve itself with respect to the valve seat and of course vice versa, can take place in a rotatable linear manner via the screw connection or otherwise.

## Claims

1. Adjustment device, in particular a volume adjustor, for adjusting the flow rate of a gas flow, for instance oxygen, which adjustment device comprises a valve seat and a valve, the valve seat and the valve being designed movable with respect to each other, the valve being provided with a protruding part and the valve seat being provided with an opening for accommodating the protruding part therein, over an adjustable distance, the valve being moveable, preferably in a linear manner, with respect to the valve seat by means of rotation, the degree of rotation defining the adjustable distance of the protruding part in the opening and an adjusted quantity of gas, and in which the adjusted quantity of the adjustment device can directly be read from the degree of rotation.

2. Adjustment device according to claim 1, **characterized in that** the adjustment device comprises a housing having a mark line, in which the valve seat can be immovably attached to the housing, in which the housing comprises an entrance and an exit for the gas flow and in that the valve is arranged in the housing in a rotatable and preferably linear movable manner.

3. Adjustment device according to claim 1 or 2, **characterized in that**, the valve has an exterior surface which is provided with a helical groove, and that an adjusting screw can be screwed through the housing so that one of its ends extends into the groove in an unsqueezing manner.

4. Adjustment device according to any one of the claims 1, 2 or 3, **characterized in that** the valve comprises a rear part protruding from the housing to which part a selector button can be attached, on which selector button a calibration has been arranged.

5. Adjustment device according to claim 3 or 4, **characterized in that** the selector button has a casing which envelops the adjusting screw which extends into the groove of the valve, so that the adjustment device cannot be taken out just like that.

6. Adjustment device according to any one of the preceding claims, **characterized in that** the adjustment device is designed in a modular manner.

7. Adjustment device according to any one of the preceding claims, **characterized in that** the adjustment device comprises an inlet cap with a filter.

8. Adjustment device according to claim 7, **characterized in that** the adjustment device comprises an O-ring between the inlet cap and the housing.

9. Adjustment device according to claim 7 or 8, **characterized in that** the adjustment device comprises an O-ring between the valve seat and the housing.

10. Adjustment device according to claim 7, 8 or 9, **characterized in that** the adjustment device comprises an O-ring between the valve and the housing above the groove in the valve and comprises an O-ring between the valve and the housing under the groove in the valve.

11. Adjustment device according to any one of the preceding claims, **characterized in that** the protruding part of the valve is conically narrowing in the direction of the opening, preferably at a gradient of 1:25 or milder, in particular approximately 1:50, the width of the end surface of the tip being some mm, preferably approximately 2 mm.

12. Adjustment device according to any one of the preceding claims, **characterized in that** the opening of the valve seat is calibrated.

13. Adjustment device according to claim 12, **characterized in that** the thickness variation of the conical protruding part is calibrated.

14. Adjustment device according to any one of the preceding claims, **characterized in that** the material of all main parts is stainless steel.
